# EUROPEAN PATENT SPECIFICATION

(11) **EP 3 479 810 B1**
(45) Date of publication and mention of the grant of the patent: **04.05.2022**
(21) Application number: 18796567.8
(22) Date of filing: 21.09.2018
(51) Int. Cl.: A61H 1/02

(54) **DEVICE FOR ASSISTING FINGER MOVEMENT**
VORRICHTUNG ZUR UNTERSTÜTZUNG VON FINGERBEWEGUNGEN
DISPOSITIF D'ASSISTANCE AU MOUVEMENT DES DOIGTS

(30) Priority: 25.09.2017 KR 20170123417
(43) Date of publication of application: 08.05.2019
(73) Proprietor: NEOFECT Co., Ltd., Yongin-si, Gyeonggi-do 16890 (KR)
(72) Inventor: PARK, Byeong Geol, Icheon-si Gyeonggi-do 17383 (KR); YOO, Kyung Hwan, Ganghwa-gun Incheon 23051 (KR); CHOI, Young Geun, Yongin-si Gyeonggi-do 16990 (KR)
(74) Representative: dompatent von Kreisler Selting Werner - Partnerschaft von Patent- und Rechtsanwälten mbB
(86) International application number: PCT/KR2018/011270
(87) International publication number: WO 2019/059723

(56) References cited:
- EP-A1- 2 417 941
- WO-A1-91/11775
- WO-A1-2008/027002
- WO-A1-2012/018159
- CN-A- 105 496 728
- JP-A- 2010 082 342
- JP-A- 2015 208 511
- KR-A- 20110 127 252
- KR-A- 20150 121 515
- KR-B1- 101 541 082
- US-A1- 2006 224 249

## Description

### Field of the Invention

Embodiments described herein relate to an apparatus for assisting in finger motion.

### Background of the Invention

Spinal cord injury (SCI), stroke, Parkinson's disease, Lou Gehrig's disease, multiple sclerosis, cerebral palsy, and the like cause patients' hands to be paralyzed and constrain the patients from carrying out various actions required for everyday life, such as holding a cup with fingers and drinking water, tooth-brushing, turning a doorknob to open the door, and the like.

In particular, stroke or Parkinson's disease causes various physical changes depending on the condition and is accompanied by, for example, paralysis of hands and contracture of fingers. If the paralysis of the hands and the contracture of the fingers are just left continually, the muscles or joints become harder, and the patient may feel pain when moving and may have difficulty in normal activities even though the nerves are restored.

Furthermore, elderly people or rehabilitation patients with low muscular strength have difficulty in joint motion, compared to healthy people, and it is practically difficult to do exercise with general exercise equipment even though exercise is actually required.

To enable patients incapable of moving their fingers normally to perform finger motion like normal people, an apparatus for assisting the patients in moving their fingers is required. WO 2012018159, WO 2008027002, EP2417941, US 2006224249, WO 9111775, and CN 105496728 disclose movement assisting device according to state of the art.

### Summary of the Invention

The embodiment provides a finger motion assisting apparatus for providing an elastic force to straighten a finger and controlling finger motion according to a degree to which a wire is pulled, thereby enabling a patient having low muscular strength or incapable of freely moving fingers due to an abnormality in a nervous system to perform finger motion like a normal person.

The technical objects are not limited to the above-mentioned ones, and the other unmentioned technical objects will become apparent to those skilled in the art from the following description.

In accordance with an aspect , there is provided an apparatus for assisting in finger motion comprising: a wearing part; a first cover extending from the wearing part to one side and for receiving a middle finger of the wearer; a first wire and a second wire configured to pull the first cover; a pulley around which the first wire and the second wire are wound; and a motor configured to rotate the pulley; a second cover extending from the wearing part to the one side and disposed adjacent to the first cover and for receiving index finger of the wearer; and a third cover extending from the wearing part to the one side and disposed adjacent to the second cover and for receiving a thumb of the wearer, wherein the first wire and the second wire are arranged from a first point of the first cover to a second point of the first cover along a direction in which the first cover extends, and characterized in that the apparatus further comprises a driving module comprising a bracket, the motor, and the pulley, and wherein the motor and the pulley are mounted on the bracket, and wherein the bracket is disposed between the second cover and the third cover.

The other detailed items are described and illustrated in the specification and the drawings.

The wires are withdrawn from the pulley, extend along the cover, surround the periphery of the cover near the distal end of the cover, extend along the cover again, and are wound around the pulley. That is, since two wires are disposed for each cover and the wires surround the periphery of the cover at the distal end of the cover, the cover is bent inward at the same time that the outer distal end of the cover is pulled by pulling the wires, thereby naturally assisting in finger motion with high traction.

Furthermore, since the rotary shaft of the motor intersects the axis arranged along the first direction and the axis arranged along the second direction at one point on a plane, twisting of the wires may be prevented. As a result, the wires may be continuously and stably pulled and released, thereby implementing accurate finger motion.

Moreover, since the first auxiliary strap and the second auxiliary strap are detachably coupled to the wearing part, it is possible to adjust stretch tension for straightening index and middle fingers, by adjusting the locations where the distal ends of the first and second auxiliary straps are attached to the wearing part. Accordingly, in the case where a wearer has ankylosis/contracture of a finger, stretch tension may be adjusted according to the degree of ankylosis/contracture, thereby implementing a neutral posture, with the finger sufficiently straightened.

In addition, since the first support strap and the second support strap are spaced apart from each other along the circumferential direction of the third cover, bending of the thumb may be ergonomically accurately implemented.

### Brief Description of Drawings

The above and other objects and features will become apparent from the following description with reference to the following figures, wherein like reference numerals refer to like parts throughout the various figures unless otherwise specified, and wherein:
FIG. 1 is a view illustrating a state before a finger motion assisting apparatus of the disclosure is worn;
FIG. 2 is a view illustrating a state after the finger motion assisting apparatus of the disclosure is worn;
FIG. 3 is a view illustrating that a first wire and a second wire are disposed in the finger motion assisting apparatus of the disclosure;
FIG. 4 is a view illustrating that a first auxiliary strap and a second auxiliary strap are disposed in the finger motion assisting apparatus;
FIG. 5 is a view illustrating a C-grip and a pinch grip of the finger motion assisting apparatus;
FIG. 6 is a view illustrating that a motor is mounted on the finger motion assisting apparatus;
FIG. 7 is a schematic view illustrating that the rotary shaft of the motor intersects an axis arranged along a first direction and an axis arranged along a second direction at one point;
FIG. 8 is a view illustrating the finger motion assisting apparatus and a remote controller;
FIG. 9 is a view illustrating a finger motion assisting apparatus according to a first modified embodiment;
FIG. 10 is a view illustrating a finger motion assisting apparatus according to an example, not forming part of the present invention;
FIG. 11 is a view illustrating a finger motion assisting apparatus according to a third modified embodiment; and
FIG. 12 is a view illustrating a finger motion assisting apparatus according to a fourth modified embodiment.

### Detailed Description of Embodiments

The above and other aspects, features and advantages of the invention will become apparent from the following description of the following embodiments given in conjunction with the accompanying drawings.

In the specification, the singular forms include plural forms unless particularly mentioned. The terms "comprises" and/or "comprising" used herein does not exclude presence or addition of one or more other elements, in addition to the aforementioned elements. Throughout the specification, the same reference numerals dente the same elements, and "and/or" includes the respective elements and all combinations of the elements. Although "first", "second" and the like are used to describe various elements, the elements are not limited by the terms. The terms are used simply to distinguish one element from other elements.

Unless otherwise defined, all terms (including technical and scientific terms) used herein have the same meaning as commonly understood by those skilled in the art. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein.

Hereinafter, exemplary embodiments will be described in detail with reference to the accompanying drawings.

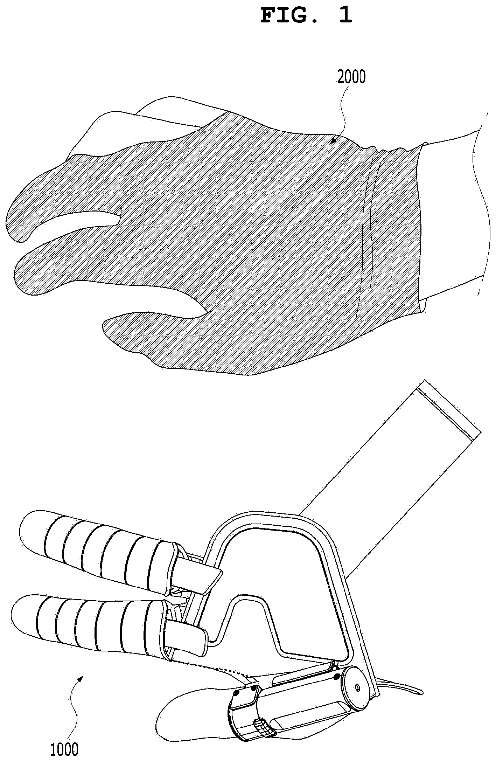

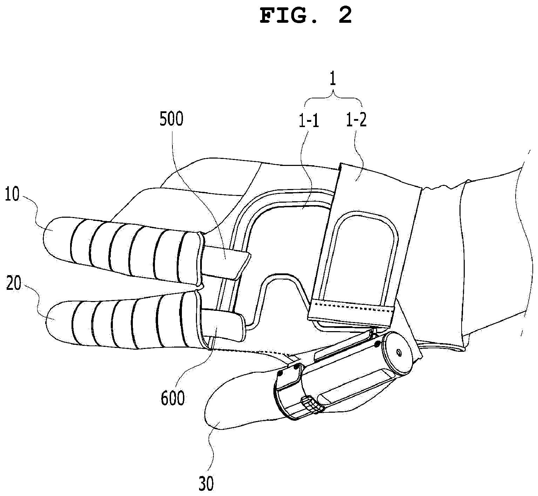

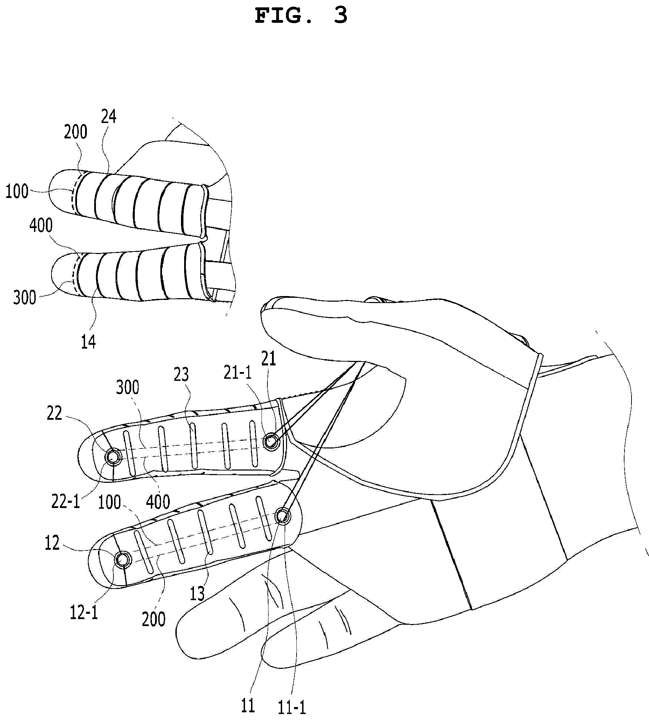

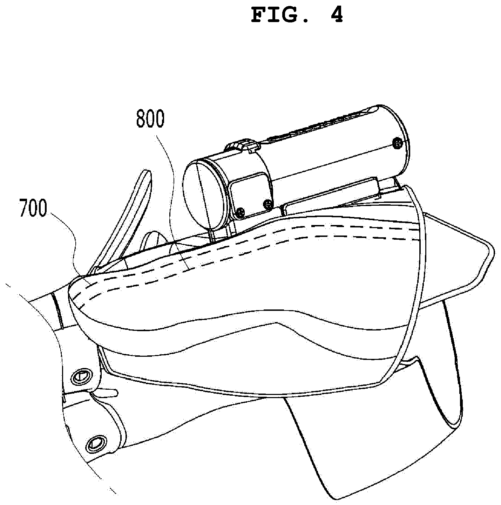

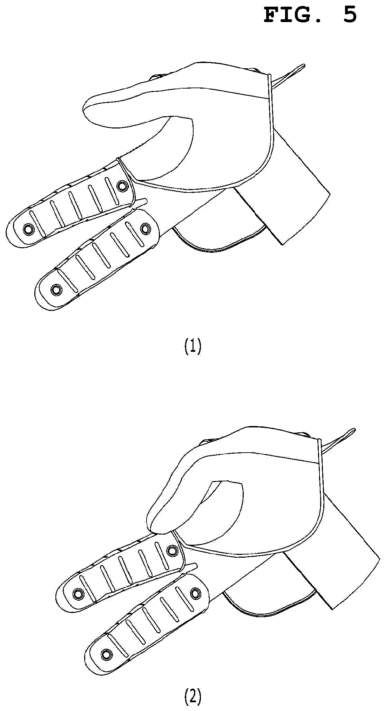

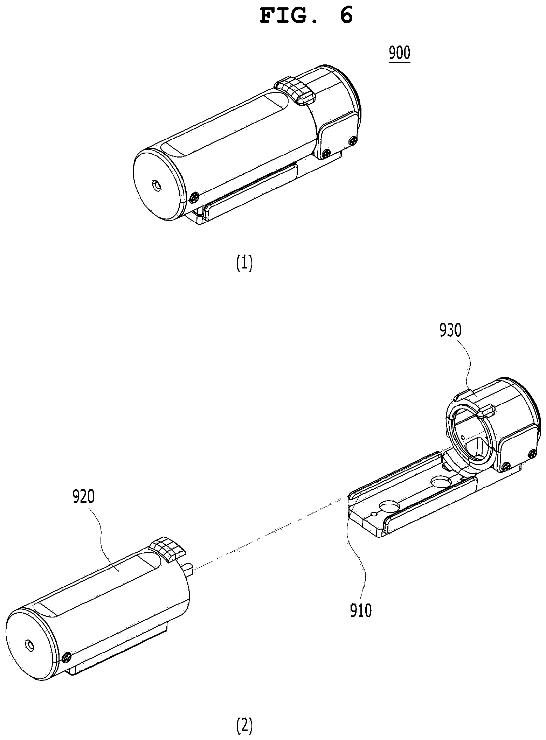

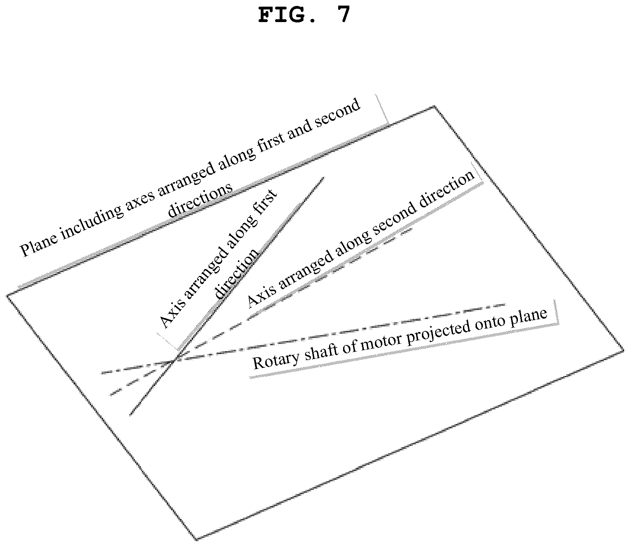

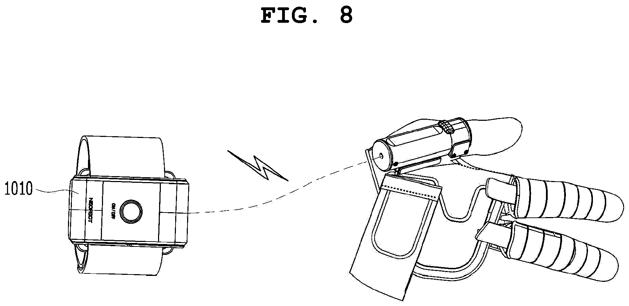

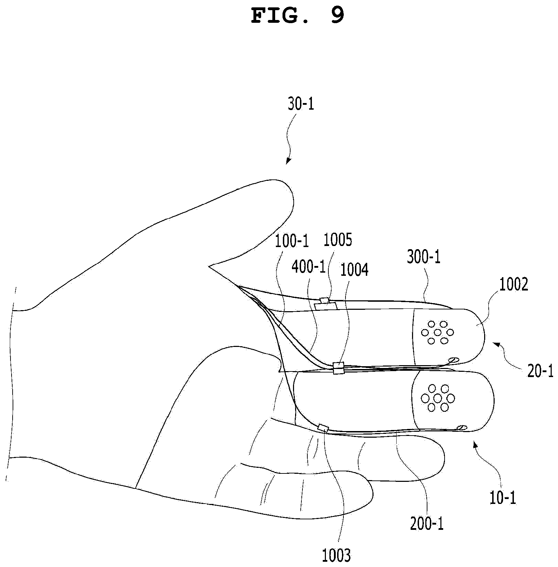

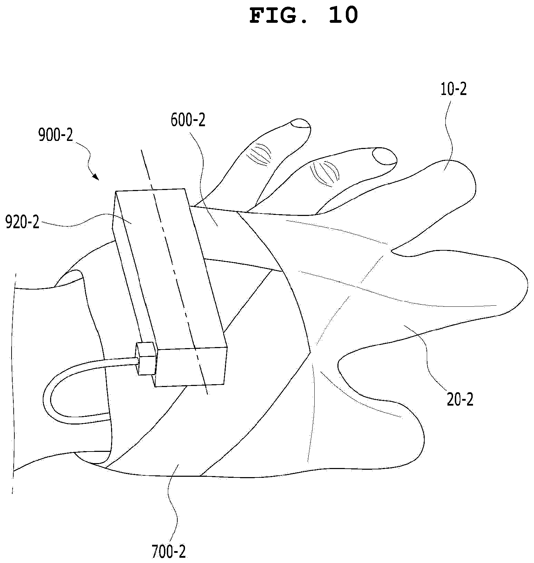

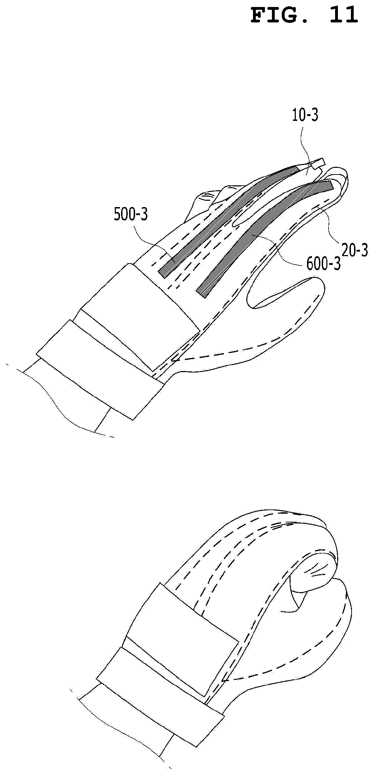

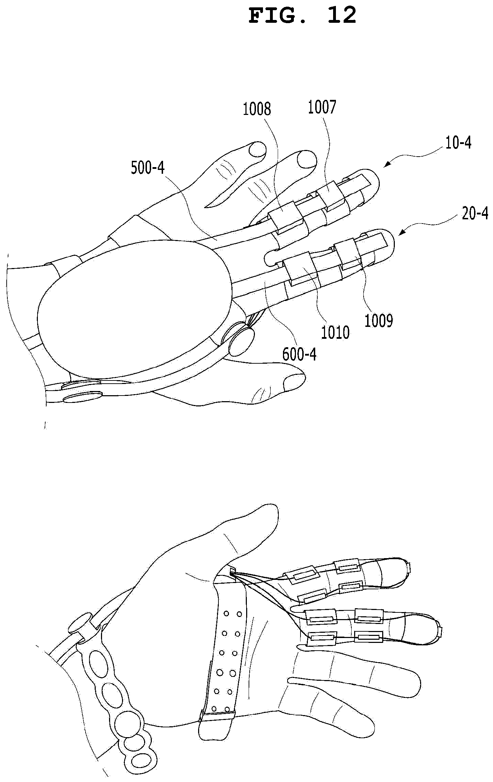

Hereinafter, a finger motion assisting apparatus 1000 will be described with reference to the accompanying drawings. FIG. 1 is a view illustrating a state before the finger motion assisting apparatus is worn. FIG. 2 is a view illustrating a state after the finger motion assisting apparatus is worn. FIG. 3 is a view illustrating that a first wire and a second wire are disposed in the finger motion assisting apparatus. FIG. 4 is a view illustrating that a first auxiliary strap and a second auxiliary strap are disposed in the finger motion assisting apparatus. FIG. 5 is a view illustrating a C-grip and a pinch grip of the finger motion assisting apparatus. FIG. 6 is a view illustrating that a motor is mounted on the finger motion assisting apparatus. FIG. 7 is a schematic view illustrating that the rotary shaft of the motor intersects an axis arranged along a first direction and an axis arranged along a second direction at one point. FIG. 8 is a view illustrating the finger motion assisting apparatus and a remote controller. FIG. 9 is a view illustrating a finger motion assisting apparatus according to a first modified embodiment. FIG. 10 is a view illustrating a finger motion assisting apparatus according to a second modified embodiment. FIG. 11 is a view illustrating a finger motion assisting apparatus according to a third modified embodiment. FIG. 12 is a view illustrating a finger motion assisting apparatus according to a fourth modified embodiment.

Hereinafter, a side corresponding to the place where the palm of a wearer's hand is located is defined as an "inside", and a side corresponding to the place where the back of the wearer's hand is located is defined as an "outside".

The finger motion assisting apparatus 1000 is an auxiliary robot of a glove type that is worn by a patient or an elderly person having difficulty in gripping or holding an object due to a finger disorder or a weak finger force to provide an auxiliary force for finger motion.

The finger motion assisting apparatus 1000 assists in motions of the index and middle fingers of the wearer by traction of wires by a motor and fixes the wearer's thumb using the rigidities of a first support strap 500 and a second support strap 600. In this case, the shape in which the thumb is bent and the degree to which the thumb is bent is modified by deforming the first support strap 500 and the second support strap 600.

The finger motion assisting apparatus 1000is an open glove through which a portion of the wearer's hand is exposed. In addition, the wearer puts on an inner glove 2000 and wears the finger motion assisting apparatus 1000over the inner glove 2000. This is intended to protect the wearer's hand and prevent slipping of the finger motion assisting apparatus 1000. In addition, at least a portion of a wearing part 1, a first cover 10, a second cover 20, or a third cover 30 may be formed of non-slip leather to more effectively prevent slipping of the finger motion assisting apparatus 1000.

The finger motion assisting apparatus 1000 includes the wearing part 1, the first cover 10, the second cover 20, the third cover 30, a first wire 100, a second wire 200, a third wire 300, a fourth wire 400, the first auxiliary strap 500, the second auxiliary strap 600, a first support strap 700, a second support strap 800, and a driving module 900.

The wearing part 1 surrounds at least part of the palm and back of the wearer's hand. The wearing part 1 may include a main body 1-1 having a Velcro formed or disposed on the outside thereof and a band 1-2 detachably coupled to the Velcro of the main body 1-1.

The first cover 10 extends from the wearing part 1 in the first direction. The wearer's middle finger is received in the first cover 10. The first cover 10 is pulled and moved by the first wire 100 and the second wire 200. As a result, the first cover 10 assists in motion of the middle finger of the wearer.

A first point 11 and a second point 12 sequentially spaced apart from each other in the extension direction of the first cover 10 is located on an inner portion of the first cover 10. A hole 11-1 in which a rivet is disposed may be formed at the first point 11 of the first cover 10, and a hole 12-1 in which a rivet is disposed may be formed at the second point 12 of the first cover 10.

The first cover 10 may have a plurality of slits 13 formed in the inner portion thereof. The plurality of slits 13 may be spaced apart from each other along the extension direction of the first cover 10. Furthermore, the first cover 10 may have a plurality of slits 14 formed in an outer portion thereof. The plurality of slits 14 may be spaced apart from each other along the extension direction of the first cover 10. The plurality of slits 13 formed in the inner portion of the first cover 10 and the plurality of slits 14 formed in the outer portion of the first cover 10 may be formed in the direction of knuckles of the middle finger received in the first cover 10. Natural bending of the actual finger may be implemented by the plurality of slits 13 formed in the inner portion of the first cover 10 and the plurality of slits 14 formed in the outer portion of the first cover 10.

The second cover 20 extends from the wearing part 1 in the second direction. The wearer's index finger is received in the second cover 20. The second cover 20 is pulled and moved by the third wire 300 and the fourth wire 400. As a result, the second cover 20 assists in motion of the index finger of the wearer.

A first point 21 and a second point 22 sequentially spaced apart from each other in the extension direction of the second cover 20 may be located on an inner portion of the second cover 20. A hole 21-1 in which a rivet is disposed may be formed at the first point 21 of the second cover 20, and a hole 22-1 in which a rivet is disposed may be formed at the second point 22 of the second cover 20.

The second cover 20 may have a plurality of slits 23 formed in the inner portion thereof. The plurality of slits 23 may be spaced apart from each other along the extension direction of the second cover 20. Furthermore, the second cover 20 may have a plurality of slits 24 formed in an outer portion thereof. The plurality of slits 24 may be spaced apart from each other along the extension direction of the second cover 20. The plurality of slits 23 formed in the inner portion of the second cover 20 and the plurality of slits 24 formed in the outer portion of the second cover 20 may be formed in the direction of knuckles of the index finger received in the second cover 20. Natural bending of the actual finger may be implemented by the plurality of slits 23 formed in the inner portion of the second cover 20 and the plurality of slits 24 formed in the outer portion of the second cover 20.

The third cover 30 extends from the wearing part 1 in a third direction. The wearer's thumb is received in the third cover 30. The first support strap 700 and the second support strap 800 may be disposed inside the third cover 300. The third cover 30 may be fixed by the rigidities of the first support strap 700 and the second support strap 800. In addition, the shape of the third cover 30 may be modified depending on the shapes of the first support strap 700 and the second support strap 800. Accordingly, the materials of the first support strap 700 and the second support strap 800 may include a deformable rigid material. For example, the materials of the first support strap 700 and the second support strap 800 may include flexible metal such as aluminum. The shape in which the thumb is bent and the degree to which the thumb is bent may be diversely implemented by deforming the shape of the third cover 30.

The first wire 100 and the second wire 200 pull the first cover 10. The first wire 100 and the second wire 200 are wound around a pulley 930 of the driving module 900.

The first wire 100 and the second wire 200 may be arranged from the first point 11 of the first cover 10 to the second point 12 of the first cover 10 along the extension direction of the first cover 10. Furthermore, the first wire 100 and the second wire 200 are arranged along at least part of the periphery of the first cover 10 in opposite directions at the second point 12 of the first cover 10.

The first wire 100 and the second wire 200 are arranged on the inner portion of the first cover 10 along the extension direction of the first cover 10. In this case, the first wire 100 and the second wire 200 sequentially pass through the hole 11-1 located at the first point 11 of the first cover 10 and the hole 12-1 located at the second point 12 of the first cover 10.

The first wire 100 and the second wire 200 are arranged along at least part of the periphery of the first cover 10 and are connected on the outer portion of the first cover 10. In this case, the first wire 100 and the second wire 200 are connected together to form a single wire (may be integrally formed with each other). Furthermore, in this case, a receiving space for receiving the first wire 100 and the second wire 200 is provided on the outer portion of the first cover 10.

As a result, when the first wire 100 and the second wire 200 are pulled, the first cover 10 pull the middle finger to bend the middle finger. In this case, owing to the above-described arrangement of the first wire 100 and the second wire 200, the first cover 10 is bent inward at the same time that the outer distal end of the first cover 10 is pulled, thereby naturally assisting in finger motion with high traction.

In the case where the first wire 100 and the second wire 200 are connected together to form a single wire, the first wire 100 and the second wire 200 are referred to as the "first cover wire".

That is, one end portion of the first cover wire 100 and 200 is wound around the pulley 930. The first cover wire 100 and 200 are withdrawn from the pulley 930, extend from the first point 11 of the first cover 10 along the extension direction of the first cover 10, are wound along at least part of the periphery of the first cover 10 at the second point 12 of the first cover 10, may return to the second point 12 of the first cover 10, and may extend to the first point 11 of the first cover 10 in the opposite direction to the extension direction of the first cover 10, and an opposite end portion of the first cover wire 100 and 200 are wound around the pulley 930.

In the case where the first cover wire 100 and 200 is wound along at least part of the periphery of the first cover 10, the first cover wire 100 and 200 may start from the inner portion of the first cover 10 and may return to the inner portion of the first cover 10 via the outer portion of the first cover 10.

The third wire 300 and the fourth wire 400 pull the second cover 20. The third wire 300 and the fourth wire 400 are wound around the pulley 930 of the driving module 900.

The third wire 300 and the fourth wire 400 are arranged from the first point 21 of the second cover 20 to the second point 22 of the second cover 20 along the extension direction of the second cover 20. Furthermore, the third wire 300 and the fourth wire 400 are arranged along at least part of the periphery of the second cover 20 in opposite directions at the second point 22 of the second cover 20.

The third wire 300 and the fourth wire 400 are arranged on the inner portion of the second cover 20 along the extension direction of the second cover 20. In this case, the third wire 300 and the fourth wire 400 sequentially pass through the hole 21-1 located at the first point 21 of the second cover 20 and the hole 22-1 located at the second point 22 of the second cover 20.

The third wire 300 and the fourth wire 400 are arranged along at least part of the periphery of the first cover 20 and are connected on the outer portion of the second cover 20. In this case, the third wire 300 and the fourth wire 400 are connected together to form a single wire. Furthermore, in this case, a receiving space for receiving the third wire 300 and the fourth wire 400 are provided on the outer portion of the second cover 20.

As a result, when the third wire 300 and the fourth wire 400 are pulled, the second cover 20 pulls the index finger to bend the index finger. In this case, owing to the above-described arrangement of the third wire 300 and the fourth wire 400, the second cover 20 is bent inward at the same time that the outer distal end of the second cover 20 is pulled, thereby naturally assisting in finger motion with high traction.

In the case where the third wire 300 and the fourth wire 400 are connected together to form a single wire, the third wire 300 and the fourth wire 400 are referred to as the "second cover wire".

That is, one end portion of the second cover wire 300 and 400 are wound around the pulley 930. The second cover wire 300 and 400 are withdrawn from the pulley 930, extend from the first point 21 of the second cover 20 along the extension direction of the second cover 20, are wound along at least part of the periphery of the second cover 20 at the second point 22 of the second cover 20, may return to the second point 22 of the second cover 20, and may extend to the first point 21 of the second cover 20 in the opposite direction to the extension direction of the second cover 20, and an opposite end portion of the second cover wire 300 and 400 are wound around the pulley 930.

In the case where the second cover wire 300 and 400 are wound along at least part of the periphery of the second cover 20, the second cover wire 300 and 400 start from the inner portion of the second cover 20 and return to the inner portion of the second cover 20 via the outer portion of the second cover 20.

The first auxiliary strap 500 may extend from the wearing part 1 into the first cover 10. In this case, the first auxiliary strap 500 may be disposed inside the first cover 10. The first auxiliary strap 500 may be coupled to the outer portion of the first cover 10. Furthermore, a portion of the first auxiliary strap 500 exposed outside the first cover 10 may be detachably coupled to the Velcro of the main body 1-1 of the wearing part 1. The material of the first auxiliary strap 500 may include an elastic material with flexibility. For example, the material of the first auxiliary strap 500 may include Neopren.

The second auxiliary strap 600 extends from the wearing part 1 into the second cover 20. In this case, the second auxiliary strap 600 is disposed inside the second cover 20. The second auxiliary strap 600 is coupled to the outer portion of the second cover 20. Furthermore, a portion of the second auxiliary strap 600 exposed outside the second cover 20 is detachably coupled to the Velcro of the main body 1-1 of the wearing part 1. The material of the second auxiliary strap 600 may include an elastic material with flexibility. For example, the material of the second auxiliary strap 600 may include Neopren.

In summary, the first auxiliary strap 500 and the second auxiliary strap 600 may straighten the first cover 10 and the second cover 20 to implement stretch tension on the index and middle fingers of the wearer. In addition, the stretch tension on the wearer's index and middle fingers may be adjusted by changing the locations where the first auxiliary strap 500 and the second auxiliary strap 600 are attached to the Velcro of the main body 1-1 of the wearing part 1. As a result, the first auxiliary strap 500 and the second auxiliary strap 600 may adjust the stretch tension according to the degree of rigidity of the wearer's fingers to implement a neutral posture in design condition.

The first support strap 700 and the second support strap 800 may be disposed inside the third cover 300. The first support strap 700 and the second support strap 800 may be disposed adjacent to each other in the circumferential direction of the third cover 30. The first support strap 700 and the second support strap 800 may be arranged in the extension direction of the third cover 30.

The first support strap 700 and the second support strap 800 may have a flat plate shape. Furthermore, as described above, the materials of the first support strap 700 and the second support strap 800 may include metal. For example, the materials of the first support strap 700 and the second support strap 800 may include aluminum. As a result, the first support strap 700 and the second support strap 800 may ensure rigidity greater than or equal to a predetermined level to fix the thumb of the wearer, and when a force greater than or equal to a predetermined level is applied, the first support strap 700 and the second support strap 800 may be deformed to implement the shape in which the thumb of a normal person is bent and the degree to which the thumb is bent.

For example, as illustrated in (1) of FIG. 5, the third cover 30 may be bent in a C-grip shape to implement a power grip, or as illustrated in (2) of FIG. 5, the third cover 30 may be bent in a pinch grip shape to implement a shape advantageous for holding a small object.

The driving module 900 may provide a driving force to pull or release the first wire 100, the second wire 200, the third wire 300, and the fourth wire 400. The driving module 900 includes a bracket 910, a motor 920, and the pulley 930.

The bracket 910 is disposed on the main body 1-1 of the wearing part 1. In this case, the bracket 910 is disposed between the second cover 20 and the third cover 30.

The motor 920 and the pulley 930 are mounted on the bracket 910. In this case, as illustrated in FIG. 6, the motor 920 is removably mounted to the pulley 930 (FIG. 6 (1) illustrates that the motor 920 is mounted to the pulley 930, and FIG. 6 (2) illustrates that the motor 920 is separated from the pulley 930).

The pulley 930 is rotated by the motor 920. The first wire 100, the second wire 200, the third wire 300, and the fourth wire 400 are wound around the pulley 930. The first wire 100, the second wire 200, the third wire 300, and the fourth wire 400 are wound around the pulley 930, or are unwound from the pulley 930 and are pulled or released, by the rotation of the pulley 930.

The rotary shaft of the motor 920 may form an acute angle with an axis arranged along the extension direction of the first cover 10 when projected onto a plane (e.g., a plane including the axis arranged in the extension direction of the first cover 10). In this case, the angle between the rotary shaft of the motor 920 and the axis arranged along the extension direction of the first cover 10 may range from 0 degrees to 80 degrees.

Likewise, the rotary shaft of the motor 920 may form an acute angle with an axis arranged along the extension direction of the second cover 20 when projected onto a plane (e.g., a plane including the axis arranged in the extension direction of the second cover 20). In this case, the angle between the rotary shaft of the motor 920 and the axis arranged along the extension direction of the second cover 20 may range from 0 degrees to 80 degrees.

Meanwhile, in the finger motion assisting apparatus 1000, the rotary shaft of the motor 920, when projected onto a plane including an axis arranged along the first direction (the extension direction of the first cover 10) and an axis arranged along the second direction (the extension direction of the second cover 20), may intersect the axis arranged along the first direction and the axis arranged along the second direction at approximately (substantially) one point.

That is, the rotary shaft of the motor 920 may intersect an extension line of the middle finger and an extension line of the index finger at the point where the extension line of the middle finger and the extension line of the index finger intersect each other. Accordingly, the first wire 100, the second wire 200, the third wire 300, and the fourth wire 400 are prevented from being twisted. As a result, the first wire 100, the second wire 200, the third wire 300, and the fourth wire 400 are continuously and stably pulled and released to implement accurate finger motion.

The finger motion assisting apparatus 1000 may further include a remote controller 1010. The remote controller 1010 may include a power button, a grip button, and a release button. The wearer may press the power button to activate the remote controller 1010 and may press the grip button to perform a grip operation. In this case, the wires are pulled by normal rotation of the motor 920. Furthermore, the wearer may press the release button to perform a release operation. In this case, the wires may be released by reverse rotation of the motor 920.

As illustrated in FIG. 8, the remote controller 1010 may be mounted in a wearable form on the wearer's arm by a band. In this case, the remote controller 1010 may be attached to or detached from the band by a magnetic force. Furthermore, the remote controller 1010 may be wirelessly or wiredly connected to the driving member 900. That is, signals of the remote controller 1010 may be transmitted to the driving module 900 through wireless communication or a conductive line.

A first modified embodiment will hereinafter be described with reference to FIG. 9. The above-described basic form may be applied to the first modified embodiment.

The first modified embodiment differs from the basic form in that the former includes a first cap 1001 and a second cap 1002 in a thimble form, and a first bracket 1003, a second bracket 1004, and a third bracket 1005 for supporting wires. Furthermore, the first modified embodiment has a feature wherein a fifth wire (not illustrated) and a sixth wire (not illustrated) are separately disposed on a third cover 30-1 (a part for receiving at least part of a thumb).

The first cap 1001 may be disposed on an end portion of a first cover 10-1 (the end portion being oriented in the extension direction of the first cover 10-1), and the second cap 1002 may be disposed on an end portion of a second cover 20-1 (the end portion being oriented in the extension direction of the second cover 20-1). The first cap 1001 may cover at least part of the end portion of the first cover 10-1, and the second cap 1002 may cover at least part of the end portion of the second cover 20-1.

A first wire 100-1 and a second wire 200-1 may be arranged on the first cap 1001 along the extension direction of the first cover 10-1. In this case, the first wire 100-1 and the second wire 200-1 may be arranged on lateral portions of the first cover 10-1 (approximately, boundary portions between an outer portion and an inner portion of the first cover 10-1) along the extension direction of the first cover 10-1 so as to be spaced apart from each other.

To this end, a first bracket 1003 may be provided on a first lateral portion of the first cover 10-1 to support the first wire 100-1, and a second bracket 1004 may be provided on a second lateral portion of the first cover 10-1 to support the second wire 200-1.

The first wire 100-1 and the second wire 200-1 may be connected together in the first cap 1001. Furthermore, the first wire 100-1 and the second wire 200-1 may be wound around a driving member (not illustrated), as in the basic form.

Likewise, a third wire 300-1 and a fourth wire 400-1 may be arranged on the second cap 1002 along the extension direction of the second cover 20-1. In this case, the third wire 300-1 and the fourth wire 400-1 may be arranged on lateral portions of the second cover 20-1 (approximately, boundary portions between an outer portion and an inner portion of the second cover 20-1) along the extension direction of the second cover 20-1 so as to be spaced apart from each other.

To this end, a third bracket 1005 may be provided on a first lateral portion of the second cover 20-1 to support the third wire 300-1, and the second bracket 1004 may be provided on a second lateral portion of the second cover 20-1 to support the fourth wire 400-1. That is, both the first wire 100-1 and the fourth wire 400-1 may be supported by the same second bracket 1004.

The third wire 300-1 and the fourth wire 400-1 may be connected together in the second cap 1002. Furthermore, the third wire 300-1 and the fourth wire 400-1 may be wound around the driving member (not illustrated), as in the basic form.

In addition, in the first modified embodiment, a fifth wire and a sixth wire may be provided on the third cover 30-1, and the third cover 30-1 may be moved by traction of the fifth and sixth wires by a motor. In this case, likewise to the first to fourth wires 100-1, 200-1, 300-1, and 400-1, the fifth and sixth wires may be wound around the driving member (not illustrated.

An example, not forming part of the present invention, will hereinafter be described with reference to FIG. 10. The above-described basic form may be applied to the example.

The example, not forming part of the present invention, differs from the basic form in terms of the form in which a first auxiliary strap 500-2 and a second auxiliary strap 600-2 are arranged and the form in which the rotary shaft of a motor 920-2 of a driving member 900-2 is arranged.

In this case, the first auxiliary strap 500-2 and the second auxiliary strap 600-2 may be arranged to cross each other. That is, the first auxiliary strap 500-2 and the second auxiliary strap 600-2 may extend to cross each other in first and second covers 10-2 and 20-2 and may be detachably coupled to the Velcro of the main body of 1-1 the wearing part 1. As a result, the wearer's index and middle fingers may be spaced apart from each other and supported by the first auxiliary strap 500-2 and the second auxiliary strap 600-2.

The rotary shaft of the motor 920-2 of the driving member 900-2, when projected onto a plane including an axis arranged along the first direction (the extension direction of the first cover 10-2) and an axis arranged along the second direction (the extension direction of the second cover 20-2), may intersect the axis arranged along the first direction and the axis arranged along the second direction at two points.

In this case, the rotary shaft of the motor 920-2 of the driving member 900-2 may be arranged such that the rotary shaft of the motor 920-2 of the driving member 900-2, the central axis of the first auxiliary strap 500-2 in the extension direction thereof, and the central axis of the second auxiliary strap 600-2 in the extension direction thereof approximately form an isosceles triangle on a plane.

A third modified embodiment will hereinafter be described with reference to FIG. 11. The above-described basic form may be applied to the third modified embodiment.

The third modified embodiment differs from the basic form in terms of the material and arrangement of a first auxiliary strap 500-3 and a second auxiliary strap 600-3.

The material of the first auxiliary strap 500-3 may include metal with high elasticity. Furthermore, the first auxiliary strap 500-3 may be disposed on the inside of an outer portion of a first cover 10-3. That is, the first cover 10-3 may have, on the outer portion thereof, a space for receiving the first auxiliary strap 500-3. To this end, the first cover 10-3 may have an auxiliary cover disposed thereon, and the space for receiving the first auxiliary strap 500-3 may be provided by sewing the auxiliary cover on the first cover 10-3.

The material of the second auxiliary strap 600-3 may include metal with high elasticity. Furthermore, the second auxiliary strap 600-3 may be disposed on the inside of an outer portion of a second cover 20-3. That is, the second cover 20-3 may have, on the outer portion thereof, a space for receiving the second auxiliary strap 600-3. To this end, the second cover 20-3 may have an auxiliary cover disposed thereon, and the space for receiving the second auxiliary strap 600-3 may be provided by sewing the auxiliary cover on the second cover 20-3.

According to the above description, in the third modified embodiment, the first cover 10-3 and the second cover 20-3 may be straightened by the first auxiliary strap 500-3 and the second auxiliary strap 600-3 (the restoring forces of the auxiliary straps) to implement stretch tension on the wearer's index and middle fingers.

A fourth modified embodiment will hereinafter be described with reference to FIG. 12. The above-described basic form may be applied to the fourth modified embodiment.

The fourth modified embodiment differs from the basic form in that the former includes a strap driving member (not illustrated) for driving a first auxiliary strap 500-4 and a second auxiliary strap 600-4 and first to fourth guides 1007 to 1010 for guiding the first auxiliary strap 500-4 and the second auxiliary strap 600-4, and the first auxiliary strap 500-4 and the second auxiliary strap 600-4 are formed of a different material.

The first auxiliary strap 500-4 and the second auxiliary strap 600-4 may be formed of a flexible material. For example, the first auxiliary strap 500-4 and the second auxiliary strap 600-4 may be thin films made of a synthetic resin.

The first auxiliary strap 500-4 may be fixed to an end portion of a first cover 10-4 (the end portion being oriented in the extension direction of the first cover 10-4), and the second auxiliary strap 600-4 may be fixed to an end portion of a second cover 20-4 (the end portion being oriented in the extension direction of the second cover 20-4).

The first auxiliary strap 500-4 and the second auxiliary strap 600-4 may be pulled and moved by the separate strap driving member (not illustrated, a motor), and the first cover 10-4 and the second cover 20-4 may be straightened by this process. Accordingly, in the fourth modified embodiment, stretch tension may be implemented on the wearer's index and middle fingers by the motor, and a neutral posture in design condition may be implemented by adjusting the stretch tension.

The first guide 1007 and the second guide 1008 may be disposed on the first cover 10-4 and may be spaced apart from each other along the extension direction of the first cover 10-4. The first auxiliary strap 500-4 may pass through the first guide 1007 and the second guide 1008 from the distal end of the first cover 10-4 and may be connected to the strap driving member. Accordingly, the first guide 1007 and the second guide 1008 may guide the moving direction of the first auxiliary strap 500-4, similarly to a rail.

Likewise, the third guide 1009 and the fourth guide 1010 may be disposed on the second cover 20-4 and may be spaced apart from each other along the extension direction of the second cover 20-4. The second auxiliary strap 600-4 may pass through the third guide 1009 and the fourth guide 1010 from the distal end of the second cover 20-4 and may be connected to the strap driving member. Accordingly, the third guide 1009 and the fourth guide 1010 may guide the moving direction of the second auxiliary strap 600-4, similarly to a rail.

The above-described embodiments are exemplary in all aspects, and should be construed not to be restrictive.

## Claims

1. An apparatus for assisting in finger motion (1000) of a wearer, the apparatus comprising:
a wearing part (1);
a first cover (10) extending from the wearing part (1) to one side and for receiving a middle finger of the wearer;
a first wire (100) and a second wire (200) configured to pull the first cover (10);
a pulley (930) around which the first wire and the second wire are wound; and
a motor (920) configured to rotate the pulley (930);
a second cover (20) extending from the wearing part (1) to the one side and disposed adjacent to the first cover (10) and for receiving index finger of the wearer; and
a third cover (30) extending from the wearing part (1) to the one side and disposed adjacent to the second cover (20) and for receiving a thumb of the wearer,
wherein the first wire(100) and the second wire(200) are arranged from a first point(11) of the first cover(10) to a second point(12) of the first cover(10) along a direction in which the first cover(10) extends, and
**characterized in that** the apparatus further comprises a driving module (900) comprising a bracket (910), the motor (920), and the pulley (930), and
wherein the motor (920) and the pulley (930) are mounted on the bracket (910), and
wherein the bracket (910) is disposed on the main body (1-1) of the wearing part (1) between the second cover (20) and the third cover (30).

2. The apparatus of claim 1, wherein the first wire (100) and the second wire (200) are arranged on an inner portion of the first cover (10) along the extension direction of the first cover (10), are arranged along the at least part of the periphery of the first cover (10), and are connected on an outer portion of the first cover (10).

3. The apparatus of claim 2, wherein a receiving space for receiving the first wire (100) and the second wire (200) is provided on the outer portion of the first cover (10), and at least part of the first wire (100) and the second wire (200) is not exposed to the outside through the outer portion of the first cover (10).

4. The apparatus of claim 1, wherein holes (11-1, 12-1) through which the first wire (100) and the second wire (200) pass are formed at the first point (11) of the first cover (10) and the second point (12) of the first cover (10), respectively.

5. The apparatus of claim 1, further comprising:
a first auxiliary strap (500) extending from the wearing part (1) into the first cover (10),
wherein the first auxiliary strap (500) is detachably coupled to the wearing part (1).

6. The apparatus of claim 1, wherein a plurality of slits (13) spaced apart from each other along the extension direction of the first cover (10) are formed in an outer portion and an inner portion of the first cover (10), and
wherein the plurality of slits (13) of the first cover (10) are formed in a direction of knuckles of a finger received in the first cover (10).

7. The apparatus of claim 1, further comprising:
a third wire (300) and a fourth wire (400) configured to pull the second cover (20),
wherein the third wire (300) and the fourth wire (400) are wound around the pulley (930), are arranged from a first point (21) of the second cover (20) to a second point (22) of the second cover (20) along a direction in which the second cover (20) extends.

8. The apparatus of claim 7, further comprising:
a first support strap (700) and a second support strap (800) disposed on the third cover (30),
wherein the first support strap (700) and the second support strap (800) are disposed adjacent to each other in a circumferential direction of the third cover (30) and are arranged along a direction in which the third cover (30) extends.

9. The apparatus of claim 1, wherein a rotary shaft of the motor (920) forms an acute angle with an axis arranged along the extension direction of the first cover (10) when projected onto a plane.

## Patentansprüche

1. Vorrichtung (1000) zur Unterstützung der Fingerbewegung eines Trägers, wobei Vorrichtung aufweist:
ein Trageteil (1);
eine erste Abdeckung (10), die sich von dem Trageteil (1) zu einer Seite erstreckt und einen Mittelfinger des Trägers aufnimmt;
einen ersten Draht (100) und einen zweiten Draht (200), die dazu ausgebildet sind, die erste Abdeckung (10) zu ziehen;
eine Rolle (930), um welche der erste und der zweite Draht gewickelt sind; und
einen Motor (920), der dazu ausgebildet ist, die Rolle (930) zu drehen;
eine zweite Abdeckung (20), die sich von dem Trageteil (1) zu der einen Seite erstreckt und der ersten Abdeckung (10) benachbart angeordnet ist und den Zeigefinger des Trägers aufnimmt; und
eine dritte Abdeckung (30), die sich von dem Trageteil (1) zu der einen Seite erstreckt und der zweiten Abdeckung (20) benachbart angeordnet ist und einen Daumen des Trägers aufnimmt,
wobei der erste Draht (100) und der zweite Draht (200) von einem ersten Punkt (11) der ersten Abdeckung (10) zu einem zweiten Punkt (12) der ersten Abdeckung (10) in einer Richtung angeordnet sind, in welche sich die erste Abdeckung (10) erstreckt, und
**dadurch gekennzeichnet, dass** die Vorrichtung ferner ein Antriebsmodul (900) mit einem Halter (910), dem Motor (920) und der Rolle (930) aufweist, und
wobei der Motor (920) und die Rolle (930) an dem Halter (910) nagebracht sind, und
wobei der Halter (910) an dem Hauptkörper (1-1) des Tragteils (1) zwischen der zweiten Abdeckung (20) und der dritten Abdeckung (30) angeordnet ist.

2. Vorrichtung nach Anspruch 1, bei welcher der erste Draht (100) und der zweite Draht (200) auf einem inneren Bereich der ersten Abdeckung (10) entlang der Erstreckungsrichtung der ersten Abdeckung (10) angeordnet sind, entlang zumindest einem Teil des Umfangs der ersten Abdeckung (10) angeordnet sind, und mit einem äußeren Bereich der ersten Abdeckung (10) verbunden sind.

3. Vorrichtung nach Anspruch 2, bei welcher ein Aufnahmeraum zur Aufnahme des ersten Drahts (100) und des zweiten Drahts (200) auf dem äußeren Bereich der ersten Abdeckung (10) vorgesehen ist, und zumindest ein Teil des ersten Drahts (100) und des zweiten Drahts (200) nicht durch den äußeren Bereich der ersten Abdeckung (10) nach außen freiliegt.

4. Vorrichtung nach Anspruch 1, bei welchem Löcher (11-1, 12-1), durch welche der erste Draht (100) und der zweite Draht (200) hindurchgehen, jeweils an dem ersten Punkt (11) der ersten Abdeckung (10) und dem zweiten Punkt (12) der ersten Abdeckung (10) gebildet sind.

5. Vorrichtung nach Anspruch 1, ferner mit:
einem ersten Hilfsgurt (500), der sich von dem Trageteil (1) in die erste Abdeckung (10) erstreckt,
wobei der erste Hilfsgurt (500) lösbar mit dem Trageteil (1) verbunden ist.

6. Vorrichtung nach Anspruch 1, bei welcher mehrere, in der Erstreckungsrichtung der ersten Abdeckung beabstandete Schlitze (13) in einem äußeren Bereich und einem inneren Bereich der ersten Abdeckung (10) ausgebildet sind, und
wobei die mehreren Schlitze (13) der ersten Abdeckung (10) in einer Richtung der Knöchel eines in der ersten Abdeckung (10) aufgenommenen Fingers ausgebildet sind.

7. Vorrichtung nach Anspruch 1, ferner mit:
einem dritten Draht (300) und einem vierten Draht (400), die dazu ausgebildet sind, die zweite Abdeckung (20) zu ziehen,
wobei der dritte Draht (300) und der vierte Draht (400) um die Rolle (930) gewickelt sind und von einem ersten Punkt ((21) der zweiten Abdeckung (20) zu einem zweiten Punkt (22) der zweiten Abdeckung (20) entlang einer Richtung angeordnet sind, in welcher sich die zweite Abdeckung (20) erstreckt.

8. Vorrichtung nach Anspruch 7, ferner mit:
einem ersten Stützgurt (700) und einem zweiten Stützgurt (800), die auf der dritten Abdeckung (30) angeordnet sind,
wobei der erste Stützgurt (700) und der zweite Stützgurt (800) einander in Umfangsrichtung der dritten Abdeckung (30) benachbart angeordnet sind und entlang einer Richtung angeordnet sind, in welcher sich die dritte Abdeckung (30) erstreckt.

9. Vorrichtung nach Anspruch 1, bei welcher, bei Projektion auf eine Ebene, eine Drehwelle des Motors (920) einen spitzen Winkel mit einer sich in Erstreckungsrichtung der ersten Abdeckung (10) angeordneten Achse bildet.

## Revendications

1. Appareil d'assistance au mouvement des doigts (1000) d'un utilisateur, l'appareil comprenant :
une partie se portant (1) ;
un premier couvercle (10) s'étendant de la partie se portant (1) jusqu'à un côté et pour recevoir un majeur de l'utilisateur ;
un premier fil (100) et un deuxième fil (200) conçus pour tirer le premier couvercle (10) ;
une poulie (930) autour de laquelle le premier fil et le deuxième fil sont enroulés ; et
un moteur (920) conçu pour faire tourner la poulie (930) ;
un deuxième couvercle (20) s'étendant de la partie se portant (1) jusqu'au côté et disposé adjacent au premier couvercle (10) et pour recevoir l'index de l'utilisateur ; et
un troisième couvercle (30) s'étendant de la partie se portant (1) jusqu'au côté et disposé adjacent au deuxième couvercle (20) et pour recevoir un pouce de l'utilisateur,
dans lequel le premier fil (100) et le deuxième fil (200) sont agencés à partir d'un premier point (11) du premier couvercle (10) jusqu'à un second point (12) du premier couvercle (10) le long d'une direction dans laquelle le premier couvercle (10) s'étend, et
**caractérisé en ce que** l'appareil comprend en outre un module d'entraînement (900) comprenant un support (910), le moteur (920) et la poulie (930), et
dans lequel le moteur (920) et la poulie (930) sont montés sur le support (910), et
dans lequel le support (910) est disposé sur le corps principal (1-1) de la partie se portant (1) entre le deuxième couvercle (20) et le troisième couvercle (30).

2. Appareil selon la revendication 1, dans lequel le premier fil (100) et le deuxième fil (200) sont agencés sur une partie intérieure du premier couvercle (10) le long de la direction d'extension du premier couvercle (10), sont agencés le long de l'au moins une partie de la périphérie du premier couvercle (10), et sont reliés sur une partie extérieure du premier couvercle (10).

3. Appareil selon la revendication 2, dans lequel un espace de réception pour recevoir le premier fil (100) et le deuxième fil (200) est prévu sur la partie extérieure du premier couvercle (10), et au moins une partie du premier fil (100) et du deuxième fil (200) n'est pas exposée à l'extérieur à travers la partie externe du premier couvercle (10).

4. Appareil selon la revendication 1, dans lequel des trous (11-1, 12-1) à travers lesquels passent le premier fil (100) et le deuxième fil (200) sont formés au niveau du premier point (11) du premier couvercle (10) et du second point (12) du premier couvercle (10), respectivement.

5. Appareil selon la revendication 1, comprenant en outre :
une première sangle auxiliaire (500) s'étendant à partir de la partie se portant (1) jusque dans le premier couvercle (10),
dans lequel la première sangle auxiliaire (500) est accouplée de manière amovible à la partie se portant (1).

6. Appareil selon la revendication 1, dans lequel une pluralité de fentes (13) espacées les unes des autres le long de la direction d'extension du premier couvercle (10) sont formées dans une partie extérieure et une partie intérieure du premier couvercle (10), et
dans lequel la pluralité de fentes (13) du premier couvercle (10) sont formées dans une direction des articulations d'un doigt reçu dans le premier couvercle (10).

7. Appareil selon la revendication 1, comprenant en outre :
un troisième fil (300) et un quatrième fil (400) conçus pour tirer le deuxième couvercle (20),
dans lequel le troisième fil (300) et le quatrième fil (400) sont enroulés autour de la poulie (930), sont agencés à partir d'un premier point (21) du deuxième couvercle (20) jusqu'à un second point (22) du deuxième couvercle (20) le long d'une direction dans laquelle le deuxième couvercle (20) s'étend.

8. Appareil selon la revendication 7, comprenant en outre :
une première sangle de support (700) et une seconde sangle de support (800) disposées sur le troisième couvercle (30),
dans lequel la première sangle de support (700) et la seconde sangle de support (800) sont disposées de manière adjacente l'une à l'autre dans une direction circonférentielle du troisième couvercle (30) et sont agencées le long d'une direction dans laquelle s'étend le troisième couvercle (30).

9. Appareil selon la revendication 1, dans lequel un arbre rotatif du moteur (920) forme un angle aigu avec un axe agencé le long de la direction d'extension du premier couvercle (10) lorsqu'il est projeté sur un plan.
